# EUROPÄISCHE PATENTANMELDUNG

(11) **EP 2 402 066 A1**
(43) Veröffentlichungstag der Anmeldung: **04.01.2012**
(21) Anmeldenummer: 11005353.5
(22) Anmeldetag: 30.06.2011
(51) Int. Cl.: B01D 21/02, C12M 1/107

(54) **Unterdruckbehälter, System und Verfahren zum Entfernen von Störstoffen aus einer Biogasanlage**

(30) Priorität: 30.06.2010 DE 102010025636
(71) Anmelder: Beutler & Lang Schalungs- und Behälterbau GmbH, 87340 Marktbreit (DE)
(72) Erfinder: Nöring, Josef, 30938 Burgwedel (DE); Christ, Hans-Wilhelm, 97199 Ochsenfurt (DE)
(74) Vertreter: Schmid, Nils T.F.

(57) **Zusammenfassung**

Ein Unterdruckbehälter (10) zum Anschließen an eine Biogansanlage, die Biogas durch Vergärung einer Biomasse erzeugt, umfassend einen Einlauf zum Anschließen an eine in die Biogasanlage ragende Absaugleitung und zum Befüllen des Unterdruckbehälters mit einer Mischung, die Störstoffe, wie Sand und eine Anlagenflüssigkeit, die Wasser enthält, umfasst, einen Auslass (5) zum Abgeben von Störstoffen, einen Ablauf (4) zum Abgeben von Anlagenflüssigkeit und zum Anschließen an eine Rückführleitung in die Biogansanlage, sowie eine Entlüftungsöffnung, über die ein Unterdruck in dem Unterdruckbehälter erzeugbar ist.

## Beschreibung

Die Erfindung betrifft einen Unterdruckbehälter, ein System und ein Verfahren zum Entfernen von Störstoffen aus einer Biogasanlage, insbesondere aus dem Fermenter einer Biogasanlage.

In einer Biogasanlage wird durch die Vergärung von organischer Biomasse Methangas erzeugt, das zur Strom- und/oder Wärmeerzeugung genutzt werden kann. Der Vergärungsprozess läuft dabei in einem abgeschlossenen Gärraum, dem Fermenter unter anaeroben Bedingungen ab. Als Ausgangsprodukt des Gärprozesses dienen in der Regel organische Rest- oder Nebenprodukte wie Flüssig- oder Festmist, Pflanzenreste, organische Schlämme, Schlachtabfälle, oder Pflanzen, wie Getreide, Zuckerrüben oder Mais.

Bei der Herstellung von Biogas werden zwei grundsätzlich verschiedene Gärverfahren eingesetzt. Einerseits die Nassfermentation, bei der das Gärsubstrat mit einem hohen Wasseranteil versetzt und während des Gärprozesses durchmischt wird. Andererseits ist auch die Trockenfermentation bekannt, die jedoch eine von der Nassfermentation unterschiedliche Zusammensetzung des Gärsubstrats erfordert. Aus der Druckschrift DE 20 2005 013 389 U1 ist eine Biogasanlage bekannt, die nach dem Nassfermentationsverfahren arbeitet. Da mit als Gärsubstrat dienenden organischen Pflanzenrohstoffen oder Wirtschaftsdünger, wie Gülle, Hühnertrockenkot oder Mist, unvermeidbar auch nicht zersetzbare Störstoffe wie Sand oder Mineralien in den Fermenter einer Biogasanlage gelangen, ist bei der bekannten Biogasanlage eine Reinigungsvorrichtung vorgesehen, die eine Mischung aus unzersetzbaren Störstoffen und Flüssigkeit an einer zentralen Position im Fermenter sammelt. Die Mischung kann auch Abbausubstanzen umfassen, die am Biogaserzeugungsprozess beteiligt sind, wie Bakterien oder Bestandteile des Gärsubstrats, die in der Flüssigkeit gelöst und/oder ungelöst in der Mischung enthalten sein können.

Von der zentralen Position kann die Mischung abgesaugt werden, um Betriebspausen der Biogasanlage wegen notwendiger Reinigungsarbeiten zu verhindern.

Bei der bekannten Biogasanlage besteht allerdings der Nachteil, dass die dem Gärprozess beim Absaugen der Störrestmischung entzogene Flüssigkeit durch die Beigabe von Flüssigkeit zum Fermentierungsprozess ersetzt werden muss, was zu einem hohen Flüssigkeitsverbrauch der bekannten Anlage beiträgt.

Darüber hinaus wird insbesondere, wenn die Anlage im mesophilen oder thermophilen Temperaturbereich betrieben wird, mit der Störstoffmischung die darin gespeicherte Wärmeenergie dem Fermenter entzogen. Dadurch ergibt sich insgesamt ein erhöhter Energiebedarf bei der bekannten Biogasanlage.

Es ist Aufgabe der Erfindung, die Nachteile des Stands der Technik zu überwinden, insbesondere einen Vakuumbehälter, ein System und ein Verfahren zum Entfernen von Störstoffen aus einer Biogasanlage bereitzustellen, durch die der Flüssigkeits- und Energieverbrauch deutlich reduziert ist.

Diese Aufgabe wird durch die Merkmale der unabhängigen Ansprüche gelöst.

Danach ist ein Unterdruckbehälter zum Anschließen an eine Biogasanlage, die Biogas durch Vergärung einer Biomasse erzeugt, vorgesehen. Der Unterdruckbehälter ist insbesondere in einer schwerkraftgemäßen Höhenrichtung oberhalb des Fermenters angeordnet. Der Unterdruckbehälter umfasst einen Einlauf zum Anschließen an eine in die Biogasanlage ragende Absaugleitung, wie ein Rohr oder einen Schlauch, und zum Befüllen des Unterdruckbehälters mit einer Mischung, die Störstoffe, wie Sand und eine Anlagenflüssigkeit, die insbesondere Wasser enthält, umfasst, einen Auslass zum Abgeben von insbesondere nicht zersetzbaren Störstoffen, einen Ablauf zum Abgeben von Anlagenflüssigkeit insbesondere in den Fermenter der Biogasanlage und eine Entlüftungsöffnung, über die ein Unterdruck in dem Unterdruckbehälter erzeugbar ist. Unter Störstoffen werden Stoffe, wie Sand, Mineralien oder Sedimente verstanden, die insbesondere im Gärprozess nicht zersetzt werden.

Vorzugsweise können der Einlauf, der Auslass, der Ablauf und die Entlüftungsöffnung über ein jeweiliges Ventil geöffnet oder geschlossen werden. Vorzugsweise sind der Einlauf, der Auslass, der Ablauf und die Entlüftungsöffnung und weitere Fluidzu- und -abläufe des Unterdruckbehälters durch jeweils eine sich in den Unterdruckbehälter erstreckende Leitungsstruktur, wie eine Rohrleitung oder einen Schlauch gebildet. Die Ventile, insbesondere pneumatisch betriebene Quetsch - und/oder Magnetventile, können in der Leitungsstruktur an einem proximalen in den Unterdruckbehälter ragenden Ende oder vorzugsweise an einem distalen aus einem Unterdruckbehälter herausragenden Abschnitt zum Öffnen und Schließen der jeweiligen Leitungsstruktur angeordnet sein. Insbesondere sind die Ventile derart aufeinander abgestimmt angesteuert, dass für einen Betriebsmodus des Unterdruckaufbaus in dem Unterdruckbehälter alle Ventile bis auf das Entlüftungsventil geschlossen sind, um den Unterdruckbehälter zu entlüften und dass für einen Abzugsbetriebsmodus zumindest das Entlüftungsventil geschlossen und das Einlaufventil geöffnet ist, so dass unter dem Einfluss des Unterdruckunterschieds zwischen dem Unterdruckbehälterinneren und dem Biogasanlageninneren die Mischung aus der Biogasanlage in den Unterdruckbehälter abgezogen wird.

Durch das Vorsehen eines Ablaufs zum Abgeben von Anlagenflüssigkeit über eine Rückführleitung in den Fermenter der Biogasanlage konnte die notwendige, zu ersetzende Wassermenge erheblich reduziert werden, wodurch der Wasserverbrauch im Vergleich zu herkömmlichen Anlagen deutlich vermindert wurde. Auch geht durch die Rückführung der Anlagenflüssigkeit in die Biogasanlage die in der Anlagenflüssigkeit gespeicherte Wärmeenergie nicht verloren, sondern wird dem Gärprozess wieder zugeführt. Da mit der Anlagenflüssigkeit auch in der Mischung vorhandene Abbausubstanzen, die den Biogaserzeugungsprozess reaktiv oder aktiv beeinflussen, zurück in die Biogasanlage ablaufen können, werden Schwankungen in der Zusammensetzung des Gärsubstrats reduziert, was einen effizienteren Anlagenbetrieb ermöglicht.

Bei einer bevorzugten Ausführung sind die Ventile des Unterdruckbehälters derart aufeinander abgestimmt angesteuert, dass für einen Betriebsmodus des Entleerens zumindest das Ablaufventil und das Auslaufventil geöffnet sind. Durch das geöffnete Ablaufventil kann die Flüssigkeit durch die Rückführleitung wieder in den Fermenter fließen. Die Rückführleitung kann durch die gleiche Leitung wie die Absaugleitung in Funktionsunion realisiert sein, ist vorzugsweise aber eine von der Absaugleitung separate Leitung oder Schlauch.

Das Auslaufventil ist insbesondere erst dann geöffnet, wenn ein Sensor, der insbesondere in Höhenrichtung unterhalb des Ablaufs, vorzugsweise benachbart dem Ablauf angeordnet ist, einen im Wesentlichen vollständigen Ablauf der Anlagenflüssigkeit ermittelt hat. Durch die Anordnung des Sensors in Höhenrichtung unterhalb und benachbart dem Ablauf kann einerseits eine maximale Menge von Anlagenflüssigkeit in den Fermenter zurückgeführt werden. Andererseits kann, wenn der Sensor Störstoffe sensiert, das Auslaufventil geöffnet werden, so dass die Störstoffe stets nicht zurück in die Biogasanlage gelangen können.

Bei einer bevorzugten Ausführung ist der Auslass des Unterdruckbehälters in Höhenrichtung unterhalb des Ablaufs angeordnet, insbesondere an einem in Höhenrichtung liegenden, unteren Ende des Unterdruckbehälters. Entsprechend der Anordnung des Störstoffauslasses am schwerkraftgemäßen Tiefpunkt des Behälters sammeln sich die wasserunlöslichen Festbestandteile der Störstoffmischung in unmittelbarer Umgebung des Auslasses im Unterdruckbehälter.

Bei einer bevorzugten Ausführung ist die Unterseite des Unterdruckbehälters im Wesentlichen kegelförmig, insbesondere mit einem Kegelspitzenwinkel größer oder gleich 60°, wobei die kegelförmige Unterseite eine gegenüber der Höhenrichtung stetig geweitete Wand bildet. Vorzugsweise ist der Auslass im Bereich eines kegelspitzen- oder kegelstumpfförmigen Endes eines Unterbereichs angeordnet. Vorzugsweise weist die kegelförmige Unterseite eine ersten, dem Auslass nahen Kegel- oder Kegelstumpfabschnitt und einen zweiten, in Höhenrichtung oberhalb an den ersten Kegelabschnitt anschließenden, zweiten kegelstumpfförmigen Kegelabschnitt auf, wobei der Kegelspitzenwinkel des ersten, dem Auslass nahen Kegelstumpfabschnitts größer als der Kegelspitzenwinkel des zweiten, dem Auslass fernen Kegelstumpfabschnitt ist. Damit flacht die Steigung der Innenwand des Unterdruckbehälters zum Auslass hin leicht ab, wodurch sich Verstopfungen im Auslassbereich vermeiden lassen.

Bei einer bevorzugten Ausführung des Unterdruckbehälters ist in Höhenrichtung zwischen dem Auslass und dem Ablauf ein Spülwassereingang zum Einbringen von Spülwasser angeordnet, wobei der Spülwassereingang durch ein Spülwassereingangsventil geöffnet und geschlossen werden kann. Das Spülwasserventil ist derart abgestimmt, dass es bei geöffnetem Auslass geöffnet wird, um die Reste mit einem Spülwasser zu vermengen.

Vorzugsweise ist der Spülwassereingang derart angeordnet, dass das Spülwasser zur Separierung der Störstoffe von den Abbausubstanzen der abgezogenen Mischung beiträgt. Vorzugsweise wird das Spülwasser dazu mit einer in Höhenrichtung nach oben weisenden Richtungskomponente eingeleitet, so dass die Abbausubstanzen im Wesentlichen nach oben gespült werden. Das Spülwasser kann auch mit einer tangential zu einer Umfangslinie der im Wesentlichen kegelförmig ausgebildeten Unterseite des Unterdruckbehälters liegenden Richtungskomponente in den Unterdruckbehälter eingebracht werden, um einen Reinigungsstrudel zu erzeugen.

Bei einer bevorzugten Weiterbildung sind das Entlüftungsventil, das Auslassventil, das Ablaufventil und das Einlaufventil pneumatisch durch eine Hauptsteuereinheit betätigt. Die Hauptsteuereinheit ist insbesondere ein Kompressor mit einer entsprechenden Steuerungs-und/oder Regelungselektronik. Vorzugsweise liegt ein Belüftungseingang, der insbesondere durch ein Belüftungsventil geöffnet oder geschlossen werden kann, in Höhenrichtung oberhalb des Einlaufs, so dass der in dem Unterdruckbehälter herrschende Unterdruck ausgeglichen werden kann.

Weiterhin betrifft die Erfindung ein System zum Entfernen von Störstoffen, wie Sand, Sedimenten oder anderen nicht zersetzbaren Stoffen, aus einer Biogasanlage, die Biogas zur Vergärung einer Biomasse erzeugt, umfassend einen erfindungsgemäßen Unterdruckbehälter mit einem Auslass zum Abgeben von einer Mischung, die Störstoffe und eine Anlagenflüssigkeit umfasst, und einer an den Auslass anschließenden Vorrichtung zum Trennen sowie insbesondere Abtransportieren von wasserunlöslichen Festbestandteilen der Mischung und Flüssigkeit.

Bei einer bevorzugten Weiterbildung des Systems ist die Separierungsvorrichtung eine Glasierschnecke. Die Glasierschnecke kann als drehbare, insbesondere motorbetriebene Förderwendel in einem Förderwendelgehäuse ausgebildet sein. Das Förderwendelgehäuse ist vorzugsweise gegenüber einer Horizontalen geneigt. Demzufolge sammelt sich die in der aus dem Auslass abgegebenen Mischung enthaltene Anlagenflüssigkeit an einem schwerpunktgemäßen Tiefpunkt in der Glasierschnecke. Die nicht zersetzbaren Störstoffe werden durch eine langsame insbesondere unterbrochene Drehung der Förderwendel abtransportiert. Vorzugsweise ist die Förderwendel intervallartig betrieben, wobei auf eine insbesondere etwa 10 Sekunden dauernde Drehbewegung eine insbesondere etwa 10 Sekunden dauernde Drehbewegungspause folgt. Bei einer bevorzugten Ausführung des Systems umfasst die Separierungsvorrichtung einen in Höhenrichtung am unteren Ende liegenden Ablass mit einem Ablassventil, über den die separierte Flüssigkeit längs einer Ablassleitung in die Biogasanlage zurückführbar ist. Vorzugsweise mündet die Ablassleitung in die Rückführleitung des Unterdruckbehälters.

Die Erfindung betrifft weiterhin ein Verfahren zum Betreiben einer Biogasanlage, die Biogas durch Vergärung einer Biomasse erzeugt, bei dem eine Biogasanlage an einen Unterdruckbehälter anschlossen wird, eine Mischung, die Störstoffe, wie an den Bestandteilen eines Gärsubstrats haftender Sand oder nicht zersetzbare Mineralien, und eine Anlagenflüssigkeit umfasst, unter dem Einfluss eines Unterdrucks in dem Unterdruckbehälter aus der Biogasanlage abzogen und in den Unterdruckbehälter eingeleitet wird, ein Auslass in dem Unterdruckbehälter geöffnet wird, um Flüssigkeit aus dem Unterdruckbehälter abzulassen insbesondere der Biogasanlage wieder zuzuführen, und insbesondere vor dem Ablassen (c) eine Absetzzeit von wenigstens 10 Sekunden, insbesondere wenigstens 30 Sekunden, vorzugsweise etwa zwei bis drei Minuten abgewartet wird, in der sich die Störstoffe von der Mischung insbesondere der Anlagenflüssigkeit vorzugsweise überwiegend absetzen können. Überwiegend bedeutet hier, dass sich etwa 50% oder mehr der Störstoffe der Mischung abgesetzt haben können.

Das Absaugen der Mischung wird insbesondere durch das Öffnen eines Einlaufventils initiiert und durch das Schließen des Einlaufventils abgeschlossen. Ein Absaugzyklus dauert insbesondere wenigstens 10 Sekunden, vorzugsweise etwa eine Minute. Der Absaugvorgang wird insbesondere einmal oder mehrere Male, vorzugsweise zwei Mal, wiederholt. Das Ablassen der von den Störstoffen getrennten Flüssigkeit aus dem Unterdruckbehälter wird insbesondere durch das Öffnen eines Ablaufventils initiiert und durch das Schließen des Ablaufventils beendet. Vorzugsweise werden beim Ablassen der Flüssigkeit insbesondere dem Rückführen der Flüssigkeit in die Biogasanlage auch in der Mischung vorhandene Abbausubtanzen abgelassen.

Vorzugsweise wird nach dem Ablassen der Flüssigkeit aus dem Druckbehälter eine Mischung, die Störstoffe und eine Anlagenflüssigkeit umfasst, insbesondere an eine anschließende Vorrichtung zum Trennen von wasserunlöslichen Festbestandteilen der Mischung und Flüssigkeit abgegeben. Die abgegebene Mischung enthält im Vergleich zu der aus der Biogasanlage abgezogenen Mischung deutlich weniger Flüssigkeit und ist dementsprechend weniger viskos. Vorzugsweise werden in der anschließenden Vorrichtung zum Trennen die wasserunlöslichen Festbestandteile und die Flüssigkeit durch eine, insbesondere langsame und intervallweise unterbrochene Drehbewegung einer Förderwendel voneinander getrennt. Vorzugsweise haften die Störstoffe unter dem Einfluss von Adhäsions- und GleitReibungskräften im Wesentlichen aneinander und an den Förderflächen, wobei die Flüssigkeit unter dem Einfluss der Schwerkraft aus der Mischung abfließen kann.

Vorzugsweise wird die Flüssigkeit nach dem Trennen in der Trennvorrichtung der Biogasanlage, insbesondere dem Fermenter, wieder zugeführt.

Vorzugsweise wird die Störstoffmischung aus dem Unterdruckbehälter nur dann abgegeben, wenn nach dem Ablassen der Flüssigkeit aus dem Unterdruckbehälter insbesondere mit einem Sensor festgestellt wurde, dass die Störstoffe ein vorbestimmtes Volumen im Unterdruckbehälter ausfüllen. Vorzugsweise werden alle dem Feststellen vorangehenden Verfahrensschritte solange wiederholt, bis die Mischung das vorbestimmte Volumen ausfüllt. Die Vorbestimmung des Volumens erfolgt insbesondere durch die Platzierung des Sensors innerhalb des Druckbehälters an einer bestimmten Höhenposition. Der Füllstand der Störstoffe kann auch durch eine Druckmesseinrichtung, wie einen Drucksensor oder ein Manometer, ermittelt werden. Gemäß der Wiederholung aller vorangehenden Verfahrensschritte kann wiederum ein-oder mehrmals verfahrensgemäß aus der Biogasanlage eine Mischung abgesaugt werden.

Vorzugsweise wird durch Öffnen eines Spülwasserventils Spülflüssigkeit mit einer vertikalen Richtungskomponente in den im Wesentlichen kegelförmigen Unterteil des Behälters eingeleitet, um die Abbausubstanzen aus der Mischung in Höhenrichtung nach oben zu spülen und im Wesentlichen von den sich im Unterteil des Behälters absetzenden Störstoffen zu trennen.

Vorzugsweise kann insbesondere der kegelförmige Unterteil des Unterdruckbehälters nach dem Abgeben der Störstoffmischung an die anschließende Trennvorrichtung durch Einleiten von Spülflüssigkeit gereinigt werden.

Vorzugsweise wird, nachdem eine Mischung aus der Biogasanlage abgesaugt worden ist, vor Beginn der Absetzzeit eine Flüssigkeit, wie Frischwasser der Biogasanlage zugeführt. Vorzugsweise wird vor Beginn des Absaugens im Unterdruckbehälter durch eine Unterdruckquelle ein Unterdruck hergestellt. Vorzugsweise bleibt die Unterdruckquelle während des Absaugvorgangs in Betrieb, so dass der Unterdruck im Unterdruckbehälter im Wesentlichen konstant gehalten wird.

Vorzugsweise wird vor dem Ende des Absaugvorgangs, insbesondere vor dem Schließen des Einlaufventils der Unterdruck in dem Behälter insbesondere durch Öffnen eines Belüftungsventils ausgeglichen. Vorzugsweise wird der letzte Absaugvorgang abgeschlossen, wenn der Unterdruck in dem Behälter ausgeglichen ist.

Vorzugsweise wird die aus der Biogasanlage, insbesondere dem Fermenter abzusaugende Mischung vor dem wenigstens einen Absaugvorgang mit Flüssigkeit angereichert. Vorzugsweise wird die aus der Biogasanlage abzusaugende Mischung zwischen aufeinander folgenden Absaugvorgängen mit Flüssigkeit angereichert.

Weitere Eigenschaften, Vorteile und Merkmale der Erfindung werden durch die folgende Beschreibung einer bevorzugten Ausführung anhand der beiliegenden Zeichnung deutlich, die zeigt:

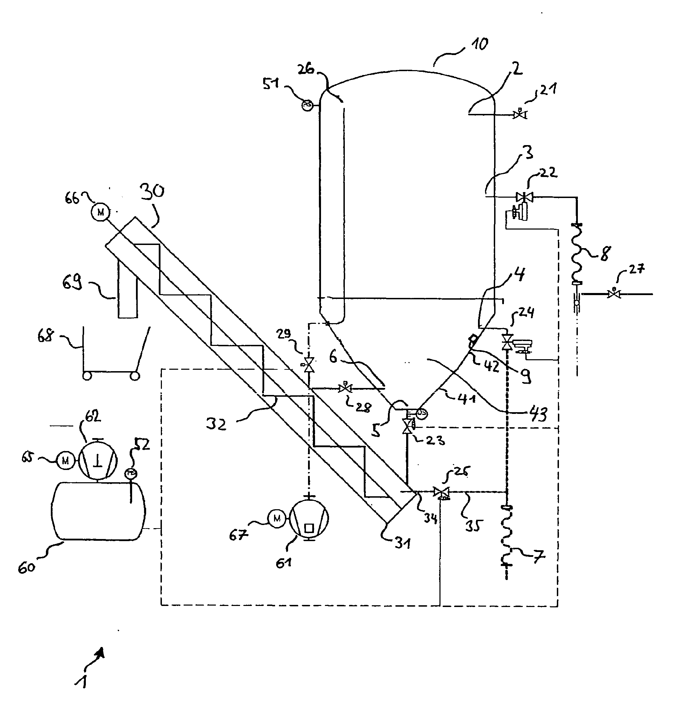

- Figur 1: Aufbau eines erfindungsgemäßen Systems zum Entfernen von Störstoffen aus einer Biogasanlage mit einem erfindungsgemäßen Unterdruckbehälter.

In Figur 1 ist ein erfindungsgemäßes System 1 zum Entfernen von Störstoffen aus einer nicht näher dargestellten Biogasanlage mit einem erfindungsgemäßen Unterdruckbehälter 10 dargestellt. Über eine Absaugleitung 8 kann aus dem nicht näher dargestellten Fermenter einer Biogasanlage eine rührfähige Störstoffmischung, die Störstoffe, eine Anlagenflüssigkeit, und Abbausubstanzen umfasst, angesaugt werden. Dazu muss im Unterdruckbehälter 10 eine Unterdruck gegenüber dem Fermenter herrschen. Dieser Unterdruck kann mit einer Vakuumpumpe 61, die von einem Motor 67 betrieben wird, in dem Unterdruckbehälter 10 erzeugt werden, sobald das Entlüftungsventil 29 die Entlüftungsöffnung 26 öffnet, die in Höhenrichtung im Oberteil des Behälters angeordnet ist.

Sobald ein Unterdruck hergestellt ist, kann durch die Absaugleitung 8 mittels Öffnung des Einlaufventils 22 die Mischung aus der Biogasanlage durch den Einlauf 3 abgezogen werden. Während des Aufbaus des Unterdrucks sind erfindungsgemäß alle weiteren mit dem Unterdruckbehälter in Verbindung stehenden Ventile außer dem Entlüftungsventil 29 geschlossen. Bereits vor dem Absaugen der Mischung aus der Biogasanlage kann durch Öffnung des Ventils 27 zusätzliche Flüssigkeit, z.B. Frischwasser, der Biogasanlage zugeführt werden.

Der Absaugvorgang durch den Einlauf 3 kann durch mehrmaliges Öffnen und Schließen des Einlaufventils 22 solange wiederholt werden, bis kein ausreichender Unterdruck in dem Unterdruckbehälter zur Verfügung steht oder eine gewünschte Menge der Mischung aus der Biogasanlage sich in dem Unterdruckbehälter befindet.

Nachdem ein oder mehrere Absaugvorgänge abgeschlossen sind, wird durch Öffnen des Belüftungsventils 21 der Unterdruck in dem Unterdruckbehälter 10 ausgeglichen. Über den Spülwassereingang 6 kann durch Öffnung des Spülwassereingangsventils 28 die Mischung in dem Unterdruckbehälter mit Flüssigkeit angereichert werden. Dabei werden durch nach oben gerichtetes Einleiten von Spülflüssigkeit Abbausubstanzen aus der abgezogenen Mischung ausgespült und von den Störstoffen im Wesentlichen getrennt.

Nach einer Wartezeit von circa zwei Minuten haben sich die Störstoffe aus der Mischung von der Flüssigkeit und den Biogasproduktionsstoffen abgesetzt. Die Festbestandteile befinden sich nach dem Absatzprozess im schwerkraftgemäßen unteren Bereich des Unterdruckbehälters. Mit einem Sensor 9 kann der Füllstand der Störstoffe im Unterdruckbehälter 10 erfasst werden. Sofern die Störstoffmenge groß genug ist, so dass der Flüssigkeitsstand wenigstens die Höhe des Ablaufs erreicht, können durch Öffnen des Ablaufventils 24 die Flüssigkeit und die Abbausubstanzen durch den Ablauf 4 und über die Rücklaufleitung 7 in den Fermenter zurückgeführt werden, wenn die störenden Bestandteile und die Abbausubstanzen der aus der Biogasanlage abgezogenen Mischung sich voneinander getrennt haben. Während des Rückführens ist auch das Einlaufventil 22 und damit der Einlauf 3 geöffnet, um einen Druckausgleich zwischen dem Fermenter und dem Unterdruckbehälter zu gewährleisten, so dass die Flüssigkeit ablaufen kann.

Nachdem die Flüssigkeit abgelaufen ist, kann erneut ein Vakuum im Unterdruckbehälter hergestellt werden und ein erneuter, gegebenenfalls mehrmaliger Absaugvorgang aus dem Fermenter beginnen.

Sofern jedoch der Sensor 9 signalisiert, dass eine vorbestimmte Menge an Feststoffen sich in dem Unterdruckbehälter abgelagert hat, wird durch Öffnen des Auslassventils 23, die Restmischung aus Festbestandteilen und Anlagenflüssigkeit durch den Auslass 5, der sich am höhengemäßen Tiefpunkt des Unterdruckbehälters 10 befindet, an die Glasierschnecke 30 abgegeben.

Um ein vollständiges Abfließen der Störstoffmischung zu erleichtern, ist die Unterseite 43 des Unterdruckbehälters 10 kegelförmig ausgebildet. Die kegelförmige Unterseite 43 ist aus einem Abschnitt 42 größerer Kegelsteigung und einen Abschnitt 41 geringerer Kegelsteigung gegliedert. Der dem Auslass 5 nahe liegende Abschnitt geringerer Kegelsteigung hat zur Folge, dass im Auslassbereich die Hangabtriebskräfte verringert sind, wodurch eine Verstopfung des Auslasses vermieden werden kann.

Zudem kann durch Öffnen des Spülventils 28 über den Spülwassereingang 6 Spülwasser in den Auslassbereich eingebracht werden, so dass sich eine Verstopfung lösen kann. Außerdem kann, nachdem die Mischung an die Glasierschnecke 30 abgegeben wurde, der Behälter durch Zuführen von Spülwasser gereinigt werden.

Sobald ein Teil der Mischung aus dem Auslass 5 an die Glasierschnecke 30 gegeben wurde, beginnt eine durch den Motor 66 betriebene Förderwendel 32 eine Drehbewegung. Die Drehbewegung erfolgt langsam und in Zeitintervallen, so dass zwischen den Förderbewegungen eine förderbewegungsfreie Zeit verbleibt. Flüssigkeit kann aus der Mischung in der Glasierschnecke innerhalb des Glasierschneckengehäuses 31 abfließen. Die Glasierschnecke 30 ist geneigt, so dass sich die Flüssigkeit in einem schwerkraftgemäßen Tiefpunkt sammelt. Die in der Glasierschnecke weitertransportierten, nicht zersetzbaren Störstoffe werden über einen Auslass 69 aus der Glasierschnecke abgegeben. Zum weiteren Abtransport der Störstoffe kann ein beweglicher Behälter 68 verwendet werden.

Die im schwerkraftgemäßen Tiefpunkt der Glasierschecke gesammelte Flüssigkeit kann, nachdem die Intervalldrehbewegung der Glasierschnecke abgeschlossen ist, über den Ablass 34 durch Öffnung des Ablassventils 25 an die Ablassleitung 35 abgegeben werden. Die Ablassleitung 35 mündet in die Rückführleitung 7, über die separierte Flüssigkeit wieder der Biogasanlage zugeführt wird. Alle Ventile des Systems 21, 22, 23, 24, 25, 27, 28 und 29 sind pneumatisch betätigbar. Das zugehörige pneumatische System umfasst ein Kompressoraggregat mit einem Druckspeicher 60 und einem durch den Motor 65 betriebenen Kompressor 62. Der Druckspeicher ist über einen Kontrollmanometer 52 überwachbar. Ebenfalls kann der im Unterdruckbehälter herrschende Unterdruck mittels eines Manometers 51 oder eines nicht näher dargestellten Drucksensors kontrolliert werden.

Die in der vorstehenden Beschreibung, der Figur und den Ansprüchen offenbarten Merkmale können sowohl einzeln als auch in beliebiger Kombination für die Realisierung der Erfindung in den verschiedenen Ausgestaltungen von Bedeutung sein.

### Bezugszeichenliste

- 1: System
- 2: Belüftungseingang
- 3: Einlauf
- 4: Ablauf
- 5: Auslass
- 6: Spülwassereingang
- 7: Rücklaufleitung
- 8: Absaugleitung
- 9: Sensor
- 10: Unterdruckbehälter
- 21: Belüftungsventil
- 22: Einlaufventil
- 23: Auslassventil
- 24: Ablaufventil
- 25: Ablassventil
- 26: Entlüftungsöffnung
- 27: Ventil
- 28: Spülwassereingangsventil
- 29: Entlüftungsventil
- 30: Glasierschnecke
- 31: Glasierschneckengehäuse
- 32: Förderwendel
- 34: Ablass
- 35: Ablassleitung
- 41: Abschnitt geringerer Kegelsteigung
- 42: Abschnitt größerer Kegelsteigung
- 43: kegelförmige Unterseite
- 51: Manometer
- 52: Kontrollmanometer
- 60: Druckspeicher
- 61: Vakuumpumpe
- 62: Kompressor
- 65, 66, 67: Motor
- 68: Behälter
- 69: Auslass

## Patentansprüche

1. Unterdruckbehälter (10) zum Anschließen an eine Biogansanlage, die Biogas durch Vergärung einer Biomasse erzeugt, umfassend:
- einen Einlauf (3) zum Anschließen an eine in die Biogasanlage ragende Absaugleitung (8) und zum Befüllen des Unterdruckbehälters (10) mit einer Mischung, die Störstoffe, wie Sand, und eine Anlagenflüssigkeit umfasst, die insbesondere Wasser enthält,
- einen Auslass (5) zum Abgeben von Störstoffen,
- einen Ablauf (4) zum Abgeben von Anlagenflüssigkeit und zum Anschließen an eine Rückführleitung (7) in die Biogansanlage,
- eine Entlüftungsöffnung (26), über die ein Unterdruck in dem Unterdruckbehälter (10) erzeugbar ist.

2. Unterdruckbehälter (10) nach Anspruch 1, umfassend ein Einlaufventil (22) zum Öffnen und Schließen des Einlaufs (3), ein Auslassventil (23) zum Öffnen und Schließen des Auslass (5), ein Ablaufventil (24) zum Öffnen und Schließen des Ablaufs (4) und ein Entlüftungsventil (29) zum Öffnen und Schließen der Entlüftungsöffnung (26), wobei insbesondere die Ventile derart aufeinander abgestimmt angesteuert sind, dass für einen Betriebsmodus des Unterdruckaufbaus in dem Unterdruckbehälter (10) alle Ventile bis auf das Entlüftungsventil (29) geschlossen sind, um den Unterdruckbehälter (10) zu entlüften und dass für einen Abzugsbetriebsmodus zumindest das Entlüftungsventil (29) geschlossen und das Einlaufventil (22) geöffnet ist, so dass unter dem Einfluss des Unterdruckunterschieds zwischen dem Unterdruckbehälterinneren und dem Biogansanlageinneren die Mischung aus der Biogansanlage in den Unterdruckbehälter (10) abgezogen wird.

3. Unterdruckbehälter (10) nach Anspruch 2, bei dem die Ventile derart aufeinander abgestimmt angesteuert sind, dass für einen Betriebsmodus des Entleerens zumindest das Ablaufventil (24) und das Auslassventil (23) geöffnet sind, wobei insbesondere das Auslassventil (23) erst dann geöffnet ist, wenn ein Sensor (9), der insbesondere in Höhenrichtung unterhalb das Ablaufs (4) vorzugsweise benachbart dem Ablauf (4) angeordnet ist, einen im Wesentlichen vollständigen Ablauf (4) der Anlagenflüssigkeit und/oder einen insbesondere durch eine Höhenposition des Sensors (9) vorbestimmten Störstofffüllstand im Unterdruckbehälter ermittelt hat.

4. Unterdruckbehälter (10) nach einem der vorstehenden Ansprüche, bei dem der Auslass (5) in Höhenrichtung unterhalb des Ablaufs (4) liegt, insbesondere an einem in Höhenrichtung liegenden unteren Ende des Unterdruckbehälters (10) und/oder bei dem ein Belüftungseingang (2), der insbesondere durch ein Belüftungsventil (21) geöffnet und geschlossen werden kann, in Höhenrichtung oberhalb des Einlaufs (3) liegt.

5. Unterdruckbehälter (10) nach einem der vorstehenden Ansprüche, dessen Unterseite (43) im Wesentlichen kegelförmig ausgebildet ist, wobei die kegelförmige Unterseite (43) eine gegenüber der Höhenrichtung stetig geweitete Wand bildet, wobei insbesondere der Auslass (5) im Bereich eines kegelspitzenförmigen Endes oder kegelstumpfförmigen Endes eines Unterbereichs angeordnet ist.

6. Unterdruckbehälter (10) nach einem der vorstehenden Ansprüche, bei dem in Höhenrichtung zwischen dem Auslass (5) und dem Ablauf (4) ein Spülwassereingang (6) zum Einbringen von Spülwasser angeordnet ist, wobei der Spülwassereingang (6) durch ein Spülwassereingangventil (28) geöffnet und geschlossen werden kann, wobei das Spülwasserventil (28) derart abgestimmt ist, dass es bei geöffnetem Auslass (5) geöffnet wird, um die Mischung mit einem Spülwasser zu vermengen, wobei insbesondere der Spülwassereingang im Unterdruckbehälter derart angeordnet ist, dass beim Vermengen der Mischung mit dem Spülwasser die Störstoffe von Abbausubstanzen in der Mischung getrennt werden.

7. Unterdruckbehälter (10) nach einem der vorstehenden Ansprüche, bei dem das Entlüftungsventil (29), das Auslassventil (23), das Ablaufventil (24) und das Einlaufventil (22) pneumatisch durch eine Hauptsteuereinheit, wie ein Kompressorkongregat, betätigt sind.

8. System zum Entfernen von Störstoffen aus einer Biogasanlage, die Biogas durch Vergärung einer Biomasse erzeugt, umfassend: einen insbesondere nach einem der vorstehenden Ansprüche ausgebildeten Unterdruckbehälter (10) mit einem Auslass (5) zum Abgeben von einer Mischung, die Störstoffe und eine Anlagenflüssigkeit umfasst, die insbesondere Wasser enthält, und einer an dem Auslass (5) anschließenden Vorrichtung zum Trennen von wasserunlöslichen Festbestandteilen der Mischung und Flüssigkeit.

9. System, bei dem die Separierungsvorrichtung eine Glasierschnecke (30) ist und/oder am in Höhenrichtung liegenden unteren Ende einen Ablass (34) mit einem Ablassventil (25) umfasst, über den die separierte Flüssigkeit längs einer Ablassleitung (35) in die Biogasanlage zurückführbar ist.

10. Verfahren zum Betreiben einer Biogasanlage, die Biogas durch Vergärung einer Biomasse erzeugt, bei dem
a) eine Biogasanlage an einen Unterdruckbehälter anschlossen wird,
b) eine Mischung, die Störstoffe und eine Anlagenflüssigkeit umfasst, die insbesondere Wasser enthält, unter dem Einfluss eines Unterdrucks in dem Unterdruckbehälter aus der Biogasanlage abzogen und in den Unterdruckbehälter eingeleitet wird,
c) ein Auslass in dem Unterdruckbehälter geöffnet wird, um Flüssigkeit aus dem Unterdruckbehälter (10) abzulassen insbesondere der Biogasanlage wieder zuzuführen;
d) insbesondere vor dem Ablassen (c) eine Absetzzeit von wenigstens 10 Sekunden abgewartet wird, in der sich die Störstoffe von der Mischung insbesondere der Anlagenflüssigkeit vorzugsweise überwiegend absetzen können.

11. Verfahren nach Anspruch 10, bei dem nach dem Ablassen der Flüssigkeit aus dem Unterdruckbehälter (10), eine Mischung, die Störstoffe und eine Anlagenflüssigkeit umfasst, insbesondere an eine anschließende Vorrichtung zum Trennen von wasserunlöslichen Festbestandteilen der Mischung und Flüssigkeit abgegeben wird,
wobei insbesondere die wasserunlöslichen Festbestandteilen und die Flüssigkeit Drehen einer Förderwendel (32) getrennt werden,
wobei insbesondere während einer insbesondere langsamen unterbrochenen Drehbewegung der Förderwendel (32) die Festbestandteile unter dem Einfluss von Haft- und Gleitreibungskräften im Wesentlichen aneinander und an den Förderflächen haften und die Flüssigkeit aus der Mischung unter dem Einfluss der Schwerkraft und auf Grund ihrer Viskosität abfließen kann,
wobei insbesondere nach dem Trennen der wasserunlöslichen Festbestandteile und der Flüssigkeit, die Flüssigkeit der Biogasanlage insbesondere dem Fermenter wieder zugeführt wird,
wobei insbesondere die Mischung, die Störstoffe und Anlagenflüssigkeit umfasst, an die Vorrichtung zum Trennen abgegeben wird, wenn nach dem Ablassen der Flüssigkeit in die Biogasanlage insbesondere mit einem Sensor (9) festgestellt wird, dass die Störstoffe ein vorbestimmtes Volumen im Unterdruckbehälter (10) ausfüllen, und/oder wenn ein vorbestimmter Druck im Unterdruckbehälter festgestellt wird,
wobei insbesondere alle dem Feststellen des Mischungsvolumens vorangehenden Verfahrensschritte wiederholt werden, wenn festgestellt wird, dass die Mischung nicht das insbesondere durch die Höhenlage des Sensors (9) im Unterdruckbehälter (10) vorbestimmte Volumen ausfüllt,
wobei insbesondere nach dem Abgeben der Mischung, die Störstoffe und Anlagenflüssigkeit umfasst, an die anschließende Trennvorrichtung, Spülflüssigkeit in den Unterdruckbehälter (10) eingeleitet wird,
wobei insbesondere Verschmutzungen oder Verstopfungen durch eine Reinigungsspülung aus dem Behälter abgetragen werden,
und/oder vor Beginn der Absetzzeit, insbesondere nach dem Absaugen, die in den Unterdruckbehälter (10) gesogene Mischung aus der Biogasanlage mit Flüssigkeit angereichert wird, wobei insbesondere unter dem Einfluss von in Höhenrichtung nach oben eingeleitetem Spülwasser Abbausubstanzen, in eine gegenüber den Störstoffen im Wesentlichen oberhalb liegende Höhenposition im Behälter gespült werden,
und/oder vor Beginn des ersten des wenigstens einen Absaugvorgangs im Unterdruckbehälter (10) durch eine Unterdruckquelle eine Unterdruck hergestellt wird, wobei insbesondere der Unterdruck im Unterdruckbehälter (10) während des Absaugens durch die Unterdruckquelle im Wesentlichen konstant gehalten wird,
und/oder vor dem Ende Absaugvorgangs der Unterdruck im Behälter durch Öffnen eines Ventils ausgeglichen wird, wobei insbesondere der Absaugvorgangs endet, wenn der Unterdruck im Behälter ausgeglichen ist,
und/oder vor dem Absaugvorgang, insbesondere zwischen mehreren Absaugvorgängen, die noch abzusaugende, Mischung insbesondere in dem Fermenter mit Flüssigkeit angereichert wird.
